# EUROPÄISCHE PATENTSCHRIFT

(11) **EP 1 357 932 B1**
(45) Veröffentlichungstag und Bekanntmachung des Hinweises auf die Patenterteilung: **15.12.2004**
(21) Anmeldenummer: 02716073.8
(22) Anmeldetag: 21.01.2002
(51) Int. Cl.: A61K 38/15, A61P 33/00

(54) **KRISTALLMODIFIKATION EINES CYCLISCHEN DEPSIPEPTIDS MIT BESSERER WIRKSAMKEIT**
CRYSTAL MODIFICATION OF A CYCLIC DEPSIPEPTIDE HAVING IMPROVED STRENGTH
MODIFICATION CRISTALLINE D'UN DEPSIPEPTIDE CYCLIQUE PRESENTANT UNE EFFICACITE AMELIOREE

(30) Priorität: 01.02.2001 DE 10104362
(43) Veröffentlichungstag der Anmeldung: 05.11.2003
(73) Patentinhaber: Bayer HealthCare AG, 51368 Leverkusen (DE)
(72) Erfinder: KALBE, Jochen, 42799 Leichlingen (DE); TRAEUBEL, Michael, 50733 Köln (DE); HARDER, Achim, 51109 Köln (DE); VON SAMSON-HIMMELSTJERNA, Georg, 30900 Mellendorf (DE)
(86) Internationale Anmeldenummer: PCT/EP2002/000541
(87) Internationale Veröffentlichungsnummer: WO 2002/066048

(56) Entgegenhaltungen:
- EP-A- 0 634 408
- EP-A- 0 872 481
- EP-A- 1 031 565

## Beschreibung

Die Erfindung betrifft die Verwendung der Kristallmodifikation I des cyclischen Depsipeptids der Formel (I) zur Herstellung von Arzneimitteln, insbesondere zur Bekämpfung von Endoparasiten.

Das cyclische Depsipeptid der Formel (I) ist bereits bekannt aus EP-A-0 634 408 (WO 93/19053).

Ebenfalls bereits bekannt ist das Auftreten dieses Wirkstoffes in vier verschiedenen Kristallmodifikationen wie sie in EP-A-1 031 565 (WO 99/24412) beschrieben sind. Die dort als Kristall (V) beschriebene Modifikation wurde zusammen mit einem neuen Herstellverfahren bereits in EP-A-0 872 481 (WO 97/02256) offenbart.

In der vorliegenden Anmeldung sollen für die verschiedenen Modifikationen die folgenden Bezeichnungen verwendet werden:
Modifikation I wird in EP-A-1 031 565 als "Crystal (III)" bezeichnet und hat einen Schmelzpunkt von 191,9°C.
Modifikation II wird in EP-A-1 031 565 als "Crystal (II)" bezeichnet und hat einen Schmelzpunkt von 182,4°C.
Modifikation III wird in EP-A-1 031 565 als "Crystal (I)" bezeichnet und hat einen Schmelzpunkt von 157,8°C.
Modifikation IV wird in EP-A-1 031 565 als "Prior Art Crystal (V)" beschrieben und hat einen Schmelzpunkt von 145,0°C;
diese Modifikation war gemäß den Angaben aus EP-A-1 031 565 bereits aus EP-A-0 872 481 bekannt.

Von den vier Modifikationen ist die Modifikation I die thermodynamisch stabilste, Modifikation II die thermodynamisch zweitstabilste, Modifikation III die thermodynamisch drittstabilste und Modifikation IV die thermodynamisch viertstabilste Modifikation.

Überraschenderweise wurde nun gefunden, dass die thermodynamisch stabilste Modifikation I von den genannten Modifikationen die größte Bioverfügbarkeit und daher die größte Wirksamkeit aufweist. Dies war für den Fachmann nicht zu erwarten: Bei diesem sehr schwer wasserlöslichen Wirkstoff sollte mit zunehmender thermodynamischer Stabilität die Löslichkeit und Bioverfügbarkeit sinken.

Die Erfindung betrifft die Verwendung des Depsipeptids der Formel (I) 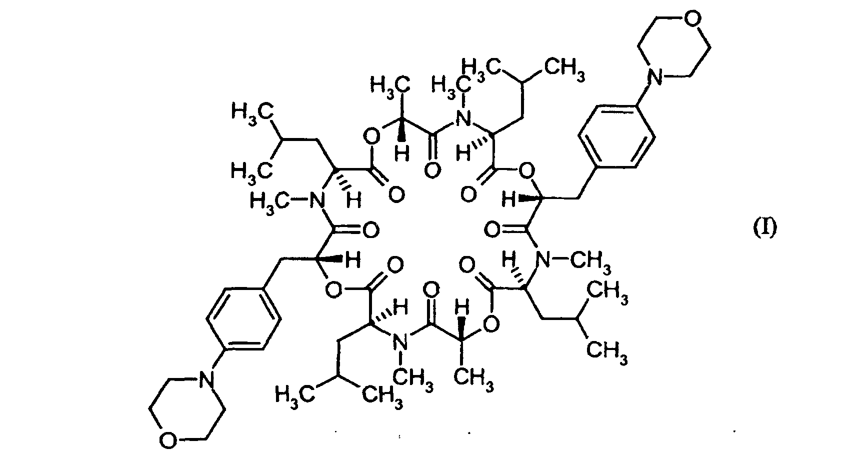 als Feststoff in der Kristallmodifikation I zur Herstellung von Arzneimitteln mit verbesserter Bioverfügbarkeit, enthaltend das Depsipeptid der Formel (I) in der Kristallmodifikation I, zur Bekämpfung von pathogenen Endoparasiten.

Aufgrund der überlegenen Bioverfügbarkeit der Modifikation I sollte das betreffende Arzneimittel einen möglichst hohen Anteil an dieser Modifikation enthalten. So sollte der Wirkstoff der Formel (I) in dem betreffenden Arzneimittel vorzugsweise zu mindestens 50 %, besonders bevorzugt mindestens 80 %, ganz besonders bevorzugt zu mindestens 90 % in der Kristallmodifikation I vorliegen. Idealerweise liegt das Depsipeptid im Arzneimittel im wesentlichen vollständig in der Modifikation I vor (d.h. mit einem Anteil von mehr als 99 %).

Erfindungsgemäß können die Arzneimittel enthaltend die Verbindung der Formel (I) in der Modifikation I eingesetzt werden zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so dass durch den Einsatz der Wirkstoffe eine wirtschaftlichere und einfachere Tierhaltung möglich ist. Vorzugsweise werden die Arzneimittel zur Bekämpfung von pathogenen Endoparasiten bei Warmblütern eingesetzt. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Parauoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spiruzida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Naturgemäß eignen sich als pharmazeutische Formulierungen erfindungsgemäß nur solche, in denen der Wirkstoff als Feststoff in der Kristallmodifikation I vorliegt. Bei Formulierungen, in denen der Wirkstoff ausschließlich in gelöster oder amorpher Form vorliegt, treten die beschriebenen Vorteile nicht ein.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, Suspensionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:

Aufgussformulierungen, Gele;

Suspensionen zur oralen oder dermalen Anwendung sowie zur Injektion; halbfeste Zubereitungen;

Formulierungen, bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen flüssigen Hilfsstoffen oder Gemischen suspendiert wird. Gegebenenfalls werden weitere Hilfsstoffe wie Farbstoffe, resorptionsfördernde Stoffe, Antioxidantien, Lichtschutzmittel, Haftmittel zugefügt.

Als flüssige Hilfsstoffe seien genannt: Wasser, Alkanole, Glycole, Polyethylenglycole, Propylenglycol, Polypropylenglycole, Glycerin, Sorbitol, Phenoxyethanol, Ester wie Essigester, Ether wie Alkylenglykolalkylether wie Dipropylenglykolmonomethylether, Diethylenglykolmono-butylether, aromatische und/oder aliphatische Kohlenwasserstoffe, pflanzliche oder synthetische Öle, 2,2-Dimethyl-4-oxy-methylen-1,3-dioxolan.

Weiterhin seien genannt: Paraffinöle, Silikonöle, natürliche Pflanzenöle wie Sesamöl, Mandelöl, Rizinusöl, synthetische Triglyceride wie Capryl /Caprinsäure-biglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, OleylalkohoL

Fettsäuren wie z.B. Ölsäure und ihre Gemische.

Farbstoffe sind alle zur Anwendung am Tier zugelassenen Farbstoffe, die gelöst oder suspendiert sein können.

Resorptionsfördemde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol, Propylgallat.

Lichtschutzmittel sind z.B. Novantisolsäure.

Haftmittel sind z.B. Cellulosederivate, Stärkederivate, Polyacrylate, natürliche Polymere wie Alginate, Gelatine.

Als weitere Hilfsstoffe seien genannt: Viskositätserhöhende und die Suspension stabilisierende Stoffe wie Carboxymethylcellulose, Methylcellulose und andere Celluloseund Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Netzmittel (Dispergiermittel) seien genannt:
Nichtionogene Tenside, z.B. polyoxyethyliertes Rizinusöl, polyoxyethyliertes Sorbitanmonooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethynolaminsalz.

Als weitere Hilfsstoffe seien die weiter oben angegebenen genannt

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden, enthalten den Wirkstoff in Konzentrationen von 0,5 - 90 Gew.-%, bevorzugt von 5 - 50 Gew.-%.

Im allgemeinen hat es sich als vorteilhaft erwiesen, Mengen von etwa 0,1 bis etwa 100 mg Wirkstoffje kg Körpergewicht pro Tag zur Erzielung wirksamer Ergebnisse zu verabreichen.

### Beispiele

### Beispiel 1: Wirksamkeit der Modifikationen I-IV des Depsipeptids der Formel (I) bei verschiedenen oralen Dosierungen gegen Haemonchus contortus im Schaf

Schafe (Merino oder Schwarzkopf Rasse, 25-35 kg Körpergewicht) wurden experimentell mit 5000 *H contortus* L3 Larven infiziert und mit der formulierten Testsubstanz am Ende der Präpatenzperiode des Parasiten behandelt. Die Testverbindungen wurden oral (Gelatinekapsel) appliziert. Die anthelminthische Wirksamkeit der Testsubstanzen wurde als Funktion der Reduktion der Eizahl pro Gramm Kot gemessen. Dazu wurde frischer Kot aus den Versuchstieren entsprechend der McMaster Methode, modifiziert nach Wetzel bearbeitet und die Eizahl ermittelt. Die Ermittlung der Eizahlen erfolgte in regelmäßigen Abständen vor und nach der Behandlung. Die anthelminthische Wirksamkeit wurde folgendermaßen definiert : 3 = >95 %, 2 = 75-95 %, 1 = 50-75 % and 0 = <50 % Eireduktion (vgl. auch G. von Samson-Himmelstjerna, A. Harder, T. Schnieder, J. Kalbe, N. Mencke (2000) In vivo activities of the new anthelmintic depsipeptide PF1022A. Parasitol. Res. 86:194-199).

| Nematode | Modifikation | Dosierung (mg/kg) | Wirksamkeit |
|---|---|---|---|
| *H. contortus* | I | 1.0 | 3 (1 Schaf, EPG) |
| *H. contortus* | I | 0.5 | 3 (1 Schaf, EPG) |
| *H. contortus* | I | 0.1 | 3 (1 Schaf, EPG) |
| *H. contortus* | II | 1.0 | 3 (1 Schaf, EPG) |
| *H. contortus* | II | 0.5 | 0 (1 Schaf, EPG) |
| *H. contortus* | II | 0.1 | 0 (1 Schaf, EPG) |
| *H. contortus* | III | 1.0 | 3 (1 Schaf, EPG) |
| *H. contortus* | III | 0.5 | 1 (1 Schaf, EPG) |
| *H. contortus* | III | 0.1 | 1 (1 Schaf, EPG) |
| *H. contortus* | IV | 1.0 | 3 (1 Schaf, EPG) |
| *H*. *contortus* | IV | 0.5 | 0 (1 Schaf, EPG) |
| *H*. *contortus* | IV | 0.1 | 0 (1 Schaf, EPG) |

3 = Wirksamkeit >95 %; 2 = Wirksamkeit 75 % bis 95 %; 1 = Wirksamkeit 50 % bis 75 %; 0 = Wirksamkeit </= 50 %; EPG = Eier pro Gramm Kot im Eireduktionstest

### Beispiel 2: Wirksamkeit der Modifikationen I und IV des Depsipeptids der Formel (I) bei verschiedenen oralen Dosierungen gegen Cooperia oncophora im Rind

Rinder (Rasse : Hostein Friesean, Art : Bos taurus, Kälber oder Jungrinder bis ca. 200 kg) wurden experimentell mit 15000 *C*. *oncophora* L3 Larven infiziert. Nach erfolgreicher Infektion (Präpatenzzeit etwa 18-21 Tage) wurden innerhalb einer Woche 3 mal die Eizahlen pro Gramm Kot ermittelt. Danach (am Tag 0 =Tag der Behandlung) wurden die Tiere mit der formulierten Testsubstanz oral (Gelatinekapsel) oder subkutan behandelt. Die anthelminthische Wirksamkeit der Testsubstanzen wurde als Funktion der Reduktion der Wurmzahl (in % der unbehandelten Kontrolle) nach Schlachtung der Tiere (10 Tage nach der Behandlung) im Dünndarm ermittelt (Vgl. auch G. von Samson-Himmelstjerna, A. Harder, T. Schnieder, J. Kalbe, N. Mencke (2000) In vivo activities of the new anthelmintic depsipeptide PF1022A. Parasitol. Res. 86 : 194-199.).

| Nematode | Modifikation | Dosierung (mg/kg) | Wirksamkeit |
|---|---|---|---|
| *C*. *oncophora* | I | 1.0 | 93.81 % (3 Rinder, Schlachtversuch) |
| *C*. *oncophora* | IV | 2.0 | 99.15 % (3 Rinder, Schlachtversuch) |
| *C. oncophora* | IV | 1.0 | 43.09 % (3 Rinder, Schlachtversuch) |

Die Wirksamkeit ist angegeben in % Wurmreduktion im Vergleich zur unbehandelten Kontrolle im Schlachtexperiment.

## Patentansprüche

1. Verwendung des Depsipeptids der Formel (I) 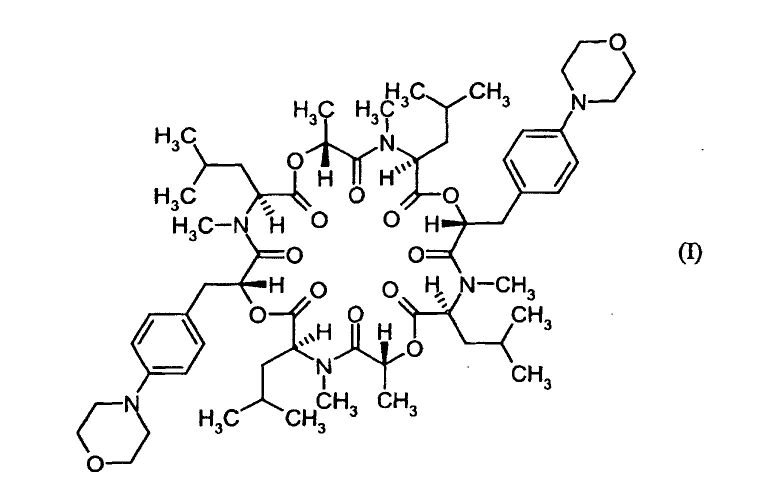 als Feststoff in der Kristallmodifikation I zur Herstellung von Arzneimitteln mit verbesserter Bioverfügbarkeit, enthaltend das Depsipeptid der Formel (I) in der Kristallmodifikation I, zur Bekämpfung von pathogenen Endoparasiten.

2. Verwendung gemäß Anspruch 1 zur Herstellung von oral verabreichbaren Arzneimitteln für Tiere.

3. Verwendung gemäß einem der Ansprüche 1 oder 2 zur Bekämpfung von Cestoden, Trematoden, Nematoden, Acantocephalen bei Tieren.

## Claims

1. Use of the depsipeptide of the formula (I) 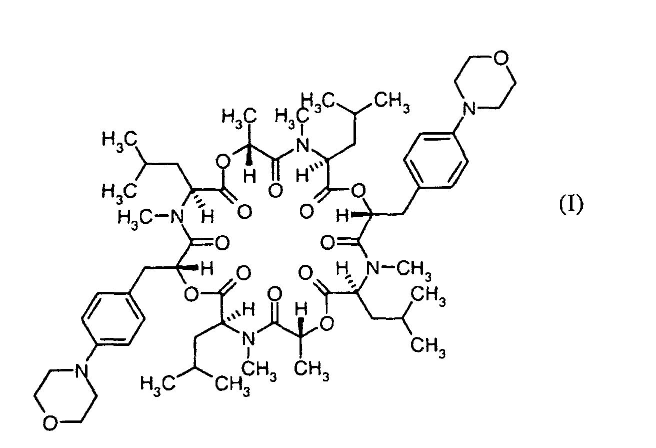 as a solid in crystal form I for the preparation of medicaments with improved bioavailability, comprising the depsipeptide of the formula (I) in crystal form I, for controlling pathogenic endoparasites.

2. Use according to Claim 1 for preparing oral medicaments for animals.

3. Use according to Claim 1 or 2 for controlling Cestodes, Trematodes, Nematodes, Acantocephala in animals.

## Revendications

1. Utilisation du depsipeptide de formule (I) 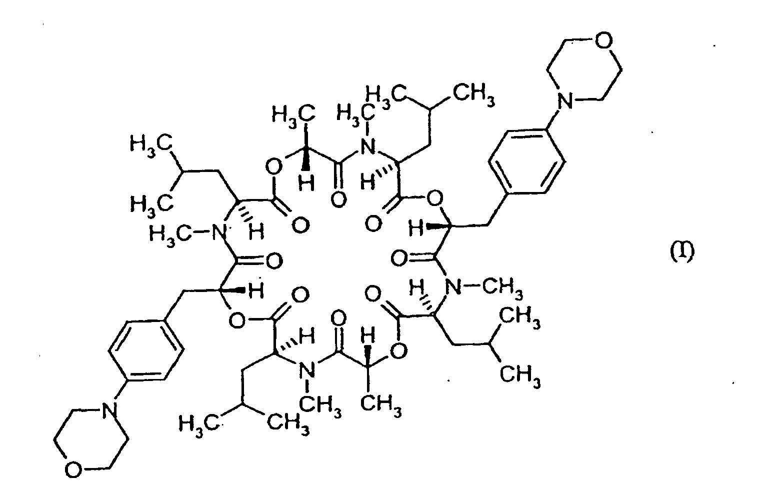 comme substance active sous la forme cristalline I pour la préparation de médicaments à biodisponibilité renforcée, contenant le depsipeptide de formule (I) sous la forme cristalline I, pour combattre des endoparasites pathogènes.

2. Utilisation suivant la revendication 1, pour la préparation de médicaments administrables par voie orale pour des animaux.

3. utilisation suivant la revendication 1 ou 2, pour combattre des cestodes, des trématodes, des nématodes, des acanthocéphales, chez des animaux.
